## Europäisches Patentamt

(19) **European Patent Office**

**Office européen des brevets**

(11) Veröffentlichungsnummer: **0 065 256**
**B1**

(12) **EUROPÄISCHE PATENTSCHRIFT**

(45) Veröffentlichungstag der Patentschrift:
03.09.86

(51) Int. Cl.⁴: **A 61 K 35/16, C 12 N 9/00**

(21) Anmeldenummer: **82104059.9**

(22) Anmeldetag: **11.05.82**

(54) Verfahren zur Herstellung von und danach hergestelltes Plasminogen.

(30) Priorität: **14.05.81 DE 3119157**

(43) Veröffentlichungstag der Anmeldung:
**24.11.82 Patentblatt 82/47**

(45) Bekanntmachung des Hinweises auf die Patenterteilung:
**03.09.86 Patentblatt 86/36**

(84) Benannte Vertragsstaaten:
**AT BE CH DE FR GB IT LI LU NL SE**

(56) Entgegenhaltungen:
**WO-A-82/03772**
**GB-A-2 068 002**

(73) Patentinhaber: **BEHRINGWERKE Aktiengesellschaft,**
**Postfach 1140, D-3550 Marburg 1 (DE)**

(72) Erfinder: **Heber, Helmut, Dr., Am Ziegenberg 8,**
**D-3550 Marburg/Lahn (DE)**
Erfinder: **Schwinn, Horst, Dr., Stümpelstal 27,**
**D-3550 Marburg/Lahn (DE)**
Erfinder: **Heimburger, Norbert, Prof. Dr.,**
**Sonnenhang 10, D-3550 Marburg/Lahn (DE)**
Erfinder: **Kumpe, Gerhardt, Dr., Perbaler Weg 11,**
**D-3552 Wetter (DE)**
Erfinder: **Schick, Manfred, Sylvester- Jordan-**
**Strasse 3, D-3550 Marburg/Lahn (DE)**

(74) Vertreter: **Meyer- Dulheuer, Karl- Hermann, Dr.,**
**HOECHST Aktiengesellschaft Zentrale**
**Patentabteilung Postfach 80 03 20, D-6230**
**Frankfurt/Main 80 (DE)**

**Beschreibung**

Die Erfindung betrifft ein Verfahren zur Herstellung praktisch hepatitisvirus-freien Human-Plasminogens durch Erwärmen in Gegenwart eines Plasmin-Inhibitors, vorzugsweise Aprotinin (Basisches Polypeptid mit polyvalenter Proteinase-Hemmwirkung) oder Soja-Bohnen-Trypsin-Inhibitor (SBTI).

Die Anmelderin hat wegen der älteren Anmeldungen GB-A-2 068 002, FR-A-2 474 313, DE-A-3 102 217 und SE 81/00393 den Gegenstand dieser Anmeldung durch die Vorlage gesonderter Patentansprüche für jene Vertragsstaaten eingeschränkt.

Die Blutgerinnung und Fibrinolyse ist ein komplexer, in Stunden ablaufender Vorgang, der durch verschiedene physiologische wie pathologische Ursachen ausgelöst wird, und dessen Ablauf von etwa 20 fördernden und hemmenden Faktoren abhängt. Durch Verminderung oder Vermehrung dieser Blutgerinnungs- und Fibrinolyse-Faktoren treten Störungen der Blutgerinnung auf, die sich teilweise als Krankheiten manifestieren. Einer dieser Faktoren ist Plasminogen.

Plasminogen enthaltende Präparate zur Behebung von Störungen, die durch einen Mangel an diesem Protein verursacht werden, sind bekannt.

Diese sind jedoch nicht frei vom Risiko einer Hepatitisübertragung.

Albumin gilt als "hepatitissicher", das heißt frei vom Risiko einer Hepatitisübertragung und damit frei von vermehrungsfähigen Hepatitisviren, wenn es in wäßriger Lösung und in Gegenwart von Stabilisatoren auf 60°C erhitzt wird (Gellis, S.S. et al., J.Clin.Invest. (1948) 27, 239).

Es ist deshalb anzunehmen, daß in Gegenwart geeigneter Stabilisatoren in wäßriger Lösung erhitztes Plasminogen ebenfalls hepatitissicher ist.

In der deutschen Offenlegungsschrift 29 16 711 ist ein Verfahren zur Stabilisierung von Plasminogen in wäßriger Lösung gegen Wärme durch Zusatz einer Aminosäure und eines Mono- oder Oligosaccharids oder Zuckeralkohols beschrieben; doch ist dieses Verfahren nur für Lösungen mit bis zu 20 CTA-Einheiten Plasminogen pro Milliliter befriedigend.

Es bestand demnach die Aufgabe, ein Verfahren zur Stabilisierung höher konzentrierter wäßriger Lösungen von Plasminogen gegen Wärme zu finden.

Überraschend wurde nun gefunden, daß eine wäßrige Lösung von Plasminogen durch Zusatz eines Proteinase-Inhibitors, vor allem eines solchen mit einer Spezifität für Plasmin, gegen Wärme stabilisiert werden kann.

Gegenstand der Erfindung ist demnach ein Verfahren zur Herstellung praktisch hepatitisvirus-freien Plasminogens durch Erwärmen einer wäßrigen Lösung von Plasminogen, die gegebenenfalls eine Aminosäure und/oder ein Saccharid oder einen Zuckeralkohols enthält, dadurch gekennzeichnet, daß die Lösung einen Proteinase-Inhibitor enthält.

Als Proteinase-Inhibitor ist beispielsweise Aprotinin oder SBTI geeignet.

Der Proteinase-Inhibitor wird in einer solchen Menge zugesetzt, daß seine Konzentration in der Lösung 0,1 bis 12,5 APE/ml bzw. 5 bis 500 KIE/ml bevorzugt 0,76 bis 2 APE/ml bzw. 30 bis 80 KIE/ml ist (APE = Antiplasmin-Einheiten; KIE = Kallikrein-Inhibition-Einheiten).

In Anwesenheit von Aprotinin oder SBTI kann die wäßrige Lösung von Plasminogen so lange erhitzt werden, daß eine Übertragung von Hepatitis-Erregern nach dem Stand des heutigen Wissens praktisch auszuschließen ist, daß also das Plasminogen keine vermehrungsfähigen Hepatitisviren enthält.

Eine Präparation, die mindestens 10 Stunden bei etwa 60° C in wäßriger Lösung gehalten wurde, gilt heute als praktisch hepatitissicher.

Eine besonders bevorzugte Ausführungsform der Erfindung ist dadurch gekennzeichnet, daß eine beispielsweise nach der deutschen Offenlegungsschrift 20 57 401 hergestellte Plasminogen enthaltende Lösung, vorzugsweise eine Plasma- oder Plazentafraktion, mit 0,1 bis 12,5 APE Aprotinin oder SBTI pro ml, vorzugsweise 0,75 bis 2 APE/ml, (5 - 500 KIE/ml, vorzugsweise 30 - 80 KIE/ml), und gegebenenfalls mit 1,0 bis 3,0 mol/l mindestens einer der Aminosäuren Glycin, $\alpha$- oder $\beta$-Alanin, Lysin, Arginin, Histidin, Hydroxyprolin, Prolin, Glutamin, einer Aminobuttersäure, vorzugsweise Glycin, und 20 bis 60% w/w eines Mono- oder Oligosaccharids oder Zuckeralkohols, vorzugsweise 1 bis 3 mol/l Glycin und 20 bis 60% w/w Saccharose, versetzt auf eine Temperatur zwischen 30 und 100° C, vorzugsweise 60 bis 100° C, erhitzt und 1 Minute bis 48 Stunden, vorzugsweise etwa 10 Stunden, bei dieser Temperatur gehalten wird, wobei die kürzeste Zeit der höchsten Temperatur zuzuordnen ist und umgekehrt. Es ist ein pH-Wert in den Grenzen zwischen 5 und 9 einzuhalten, vorzugsweise 6,5.

Es wird eine praktisch hepatitissichere Präparation von Plasminogen erhalten.

Abhängig von der Löslichkeit der Aminosäure oder des Kohlenhydrates kann die Konzentration auf über 3 mol/l bzw. 60 Gew.% erhöht werden, wenn diese bei der gewünschten Temperatur eine entsprechend höhere Löslichkeit aufweisen. Die Temperaturbehandlung kann auch in mehreren Schritten durchgeführt werden.

Mit der bevorzugt verwendeten Kombination von Aprotinin mit Glycin und Saccharose wird unter folgenden Bedingungen durch Erhitzen ein hepatitissicheres Plasminogen-Präparat erzielt: 10 bis 20 Stunden Erhitzen der Plasminogen-Lösung auf 60 bis 70°C, die 0,75 bis 2 APE/ml (30 bis 80 KIE/ml) Aprotinin, 20 bis 60 Gew.% Saccharose und 1 bis 3 mol/l Glycin enthält bei einen pH-Wert von 6 bis 7.

Wie die Tabelle zeigt, wird das Plasminogen in Lösung durch Aprotinin gegen die Einwirkung von Wärme (10 h, 60°C) stabilisiert:

| Stabilisatoren | Plasminogen (E/ml) | |
| --- | --- | --- |
| | vor Erhitzen | nach Erhitzen |
| Saccharose 60% w/w Glycin 2 mol/l | 70 CTA*/ml | 8 CTA/ml |
| Aprotinin 50 KIE/ml Saccharose 60% w/w Glycin 2 mol/l | 70 CTA/ml | 67 CTA/ml |

\* CTA-Units: Committee on Thrombolytic Agents
A.J. Johnson, D.L. Kline, N. Alkjaersig
Thromb. Diath. Haemorrh. <u>21</u>, 259 (1969)

Das Plasminogen kann aus der erhitzen Lösung durch Adsorption an Lysin-MPT-Adsorbens, Waschung und Elution, gemäß der deutschen Offenlegungsschrift 20 57 401 gereinigt werden, wodurch überschüssiges Aprotinin abgetrennt wird.

Zweckmäßigerweise geht man von Fraktionen aus, in denen Plasminogen angereichert ist, wie beispielsweise in der DOS 20 57 401 beschrieben.

Die Kontrolle der Maßnahmen zur Anreicherung und Reinigung von Plasminogen ist dem Fachmann durch die Kenntnis von Bestimmungsmethoden für Plasminogen geläufig. Unter Verwendung dieser Kontrollmethoden können die Verfahrensbedingungen unter dem Gesichtspunkt einer befriedigenden Ausbeute und einer befriedigenden Reinheit des Produktes geienkt werden.

Plasminogen kann beispielsweise nach dem von Jacobi et al. in Die Medizinische Welt 26, 1696 (1975) beschriebenen Verfahren bestimmt werden.

Zur Abtötung der Hepatitis-Viren werden der Plasminogen-Lösung Aprotinin und Glycin und Saccharose zugesetzt und erhitzt. Für die Weiterreinigung wird die erhitzte Lösung gegebenenfalls zentrifugiert. Der Überstand wird an Lysin-MPT-Adsorbens adsorbiert, das beladene Adsorbens gewaschen und mit lysinhaltigem Puffer eluiert. Eine nach diesem Verfahren erhältliche hepatitissichere Plasminogen-Präparation stellt im besonderen den Gegenstand der Erfindung dar. Zur Erhöhung der Lagerstabilität ist es zweckmäßig, der Präparation Protein-stabilisierende Substanzen, beispielsweise Proteine, Aminosäuren oder Kohlenhydrate, zuzusetzen. Schließlich kann das dieser Behandlung unterzogene Präparat in gefriergetrockneter Form latinehydrolysaten, beispielsweise Haemaccel (R), vorteilhaft sein.

Das erfindungsgemäße Produkt ist ein Mittel für die therapeutische Substitution von Plasminogen bei Patienten, bei denen eine Plasminogenwerarmung nachweisbar ist. nogen-Streptokinase-Komplexes für die Thromboysetherapie verwendet werden.

Die Erfindung soll an den nachstehenden Beispielen näher erläutert werden:

**Beispiel 1**

Herstellung eines hepatitissicheren Plasminogen-Konzentrates aus humanem Citratplasma:
0,5 l Human-Plasminogen-Lösung mit 110 CTA-Einheiten Plasminogen/ml werden mit 3 ml Aprotininlösung mit einem Gehalt von 10 000 KIE/ml versetzt. Dann werden 0,5 kg Saccharose und 75 g Glycin zugegeben und die Lösung 10 Stunden auf 60°C erhitzt.

**Beispiel 2**

1 l Plasminogen-Konzentrat mit einem Gehalt von 90 CTA-Einheiten Plasminogen pro ml wird mit 5 ml Aprotininlösung mit mit einem Gehalt von 10 KIE/ml versetzt. Anschliessend werden 1 kg Saccharose und 150 g Glycin darin aufgelöst und die Lösung 10 Stunden auf 60°C erhitzt.

**Patentansprüche** für die Vertragsstaaten GB, FR, DE und SE:

1. Verfahren zur Herstellung praktisch hepatitisvirusfreien Plasminogens durch Erwärmen einer Lösung von Plasminogen, die gegebenenfalls eine Aminosäure mit der Ausnakme von Lysin und/oder ein Saccharid oder einen Zuckeralkohol enthält, dadurch gekennzeichnet, daß die Lösung einen Proteinase-Inhibitor mit Plasmin-Spezifität enthält, wobei dieser Inhibitor nicht Aprotinin oder Trypsin-Inhibitor aus Sojabohnen ist.

2. Verfahren zur Herstellung praktisch hepatitisvirusfreien Plasminogens durch Erwärmen einer Lösung von Plasminogen, die eine Aminosäure und ein Saccharid oder einen Zuckeralkohol enthält, dadurch gekennzeichnet, daß die Lösung einen Proteinase-Inhibitor mit Plasmin-Spezifität enthält.

3. Verfahren nach Anspruch 2, dadurch gekennzeichnet, daß der Proteinase-Inhibitor Aprotinin ist.

4. Verfahren nach Anspruch 3, dadurch gekennzeichnet, daß das Aprotinin in einer Konzentration von 0,1-12,5 APE/ml (5 - 500 KIE/ml) Plasminogenlösung vorhanden ist.

5. Verfahren nach Anspruch 3, dadurch gekennzeichnet, daß Aprotinin und 1 bis 3 mol/l mindestens einer der Aminosäuren Glycin, alpha- oder beta-Alanin, Lysin, Arginin, Histidin, Hydroxyprolin, Prolin, Glutamin oder eine Aminobuttersäure und 20 bis 60 % w/w eines Mono- oder Oligosaccharids oder Zuckeralkohols zugesetzt und 1 Minuté bis 48 Stunden auf eine Temperatur von 30 bis 100°C erhitzt wird.

**Patentansprüche** für die Vertragsstaaten AT, BE, CH, IT, LI, LU, NL

1. Verfahren zur Herstellung praktisch hepatitisvirusfreien Plasminogens durch Erhwärmen einer Lösung von Plasminogen, die gegebenenfalls eine Aminosäure und/ oder ein Saccharid oder einen Zuckeralkohol enthält, dadurch gekennzeichnet, daß die Lösung einen proteinasen-Inhibitor mit Plasmin-Spezifität enthält.

2. Verfahren nach Anspruch 1, dadurch gekennzeichnet, daß der Proteinase-Inhibitor Aprotinin ist.

3. Verfahren nach Anspruch 2, dadurch gekennzeichnet, daß das Aprotinin in einer Konzentration von 0,1 - 12,5 APE/ml (5 - 500 KIE/ml) Plasminogenlösung vorhanden ist.

4. Verfahren nach Anspruch 1, dadurch gekennzeichnet, daß Aprotinin und gegebenenfalls 1 bis 3 mol/l mindestens einer der Aminosäuren Glycin, α- oder β-Alanin, Lysin, Arginin, Histidin, Hydroxyprolin, Prolin, Glutamin oder eine Aminobuttersäure und 20 bis 60% w/w eines Mono- oder Oligosaccharids oder Zuckeralkohols, zugesetzt und 1 Minute bis 48 Stunden auf eine Temperatur von 30 bis 100°C erhitzt wird.

**Claims** for the contracting states: GB, FR, DE and SE:

1. A process for the preparation of plasminogen which is virtually free of hepatitis virus by heating a solution of plasminogen, which optionally contains an aminoacid with the exception of lysine and/or a saccharide or a sugar alcohol, wherein the solution contains a proteinase inhibitor with plasmin specificity, whereby that inhibitor is not aprotinin or trypsin-inhibitor from soybeans.

2. A process for the preparation of plasminogen which is virtually free of hepatitis virus by heating a solution of plasminogen, which solution contains an aminoacid and a saccharide or a sugar alcohol, wherein the solution contains a proteinase inhibitor with plasmin specificity.

3. The process as claimed in claim 2, wherein the proteinase inhibitor is aprotinin.

4. The process as claimed in claim 3, wherein the aprotinin is present in a concentration of 0.1 - 12.5 API/ml (5-500 KIU/ml) of plasminogen solution.

5. The process as claimed in claim 3, wherein the aprotinin and 1 to 3 mole/l of at least one of the aminoacids comprising glycine, α or β-alanine, lysine, arginine, histidine, hydroxyproline, proline, glutamine or an aminobutyric acid and 20 to 60 % w/w of a mono- or oligosaccharide or sugar alcohol are added and the solution is heated to a temperature of 30 to 100°C for 1 minute to 48 hours.

**Claims** for the contracting states: AT, BE, CH, IT, LI, LU and NL:

1. A process for the preparation of plasminogen which is virtually free of hepatitis virus by heating a solution of plasminogen, which optionally contains an aminoacid and/or a saccharide or a sugar alcohol, wherein the solution contains a proteinase inhibitor with plasmin specificity.

2. The process as claimed in claim 1, wherein the proteinase inhibitor is aprotinin.

3. The process as claimed in claim 2, wherein the aprotinin is present in a concentration of 0.1 - 12.5 API/ml (5-500 KIU/ml) of plasminogen solution.

4. The process as claimed in claim 1, wherein aprotinin and optionally 1 to 3 mole/l of at least one of the aminoacids comprising glycine, α- or β-alanine, lysine, arginine, histidine, hydroxyproline, proline, glutamine or an aminobutyric acid and 20 to 60 % w/w of a mono- or oligosaccharide or sugar alcohol are added and the solution is heated to a temperature of 30 to 100°C for 1 minute to 48 hours.

**Revendications** pour les Etats contractants: GB, FR, DE et SE

1. Procédé pour la préparation de plasminogène pratiquement exempt de virus de l'hépatite, par chauffage d'une solution de plasminogène, qui contient éventuellement un acide aminé à l'exception de la lysine et/ou un saccharide ou un alcool de sucre, caractérisé en ce que la solution contient un inhibiteur de protéinase spécifique vis-à-vis de la plasmine, cet inhibiteur n'étant ni l'aprotinine, ni un inhibiteur de trypsine provenant de graines de soja.

2. Procédé pour la préparation de plasminogène pratiquement exempt de virus de l'hépatite, par chauffage d'une solution de plasminogène, qui contient un acide aminé et un saccharide ou un alcool de sucre, caractérisé en ce que la solution contient un inhibiteur de protéinase spécifique vis-à-vis de la plasmine.

3. Procédé selon la revendication 2, caractérisé en ce que l'inhibiteur de protéinase est l'aprotinine.

4. Procédé selon la revendication 3, caractérisé en ce que l'aprotinine est présente en une concentration de 0,1 à 12,5 APE/ml (de 5 à 500 KIE/ml) de solution de plasminogène.

5. Procédé selon la revendication 3, caractérisé en ce qu'on ajoute l'aprotinine et de 1 à 3 moles/l d'au moins un des acides aminés suivants: la glycine, l'alpha- ou béta-alanine, la lysine, l'arginine, l'histidine, l'hydroxyproline la proline, la glutamine ou un acide aminobutyrique, et de 20 à 60 % en poids/poids d'un mono- ou oligosaccharide ou d'un alcool de sucre, et on chauffe pendant de 1 minute à 48 heures à une température de 30 à 100°C.


**Revendications** pour les Etats contractants: AT, BE, CH, IT,LI, LU et NL

1. Procédé pour la préparation de plasminogène pratiquement exempt de virus de l'hépatite, par chauffage d'une solution de plasminogène, qui contient éventuellement un acide aminé et/ou un saccharide ou un alcool de sucre, caractérisé en ce que la solution contient un inhibiteur de protéinases spécifique vis-à-vis de la plasmine.

2. Procédé selon la revendication 1, caractérisé en ce que l'inhibiteur de protéinase est l'aprotinine.

3. Procédé selon la revendication 2, caractérisé en ce que l'aprotinine est présente en une concentration de 0,1 à 12,5 APE/ml (de 5 à 500 KIE/ ml) de solution de plasminogène.

4. Procédé selon la revendication 1, caractérisé en ce qu'on ajoute l'aprotinine et éventuellement de 1 à 3 moles/l d'au moins un des acides aminés suivants: la glycine, l'alpha- ou la béta-alanine, la lysine, l'arginine, l'histidine, l'hydroxyproline, la proline, la glutamine ou un acide amino-butyrique, et de 20 à 60 % en poids/poids d'un mono- ou oligosaccharide ou d'un alcool de sucre et on chauffe pendant de 1 minute à 48 heures à une température de 30 à 100°C.